# EUROPÄISCHE PATENTSCHRIFT

(11) **EP 2 498 725 B1**
(45) Veröffentlichungstag und Bekanntmachung des Hinweises auf die Patenterteilung: **23.10.2013**
(21) Anmeldenummer: 10782547.3
(22) Anmeldetag: 12.11.2010
(51) Int. Cl.: A61F 2/64, A61F 2/68

(54) **VORRICHTUNG UND VERFAHREN ZUR STEUERUNG EINES KÜNSTLICHEN ORTHETISCHEN ODER PROTHETISCHEN GELENKES**
DEVICE AND METHOD FOR CONTROLLING AN ARTIFICIAL ORTHETIC OR PROSTHETIC JOINT
DISPOSITIF ET PROCÉDÉ POUR COMMANDER UNE ARTICULATION ORTHÉTIQUE OU PROTHÉTIQUE ARTIFICIELLE

(30) Priorität: 13.11.2009 DE 102009052890
(43) Veröffentlichungstag der Anmeldung: 19.09.2012
(73) Patentinhaber: Otto Bock Healthcare Products GmbH, 1070 Wien (AT)
(72) Erfinder: SEYR, Martin, A-1040 Wien (AT); KAMPAS, Philipp, A-1020 Wien (AT); ZARLING, Sven, 37115 Duderstadt (DE)
(74) Vertreter: Stornebel, Kai
(86) Internationale Anmeldenummer: PCT/EP2010/006892
(87) Internationale Veröffentlichungsnummer: WO 2011/057791

(56) Entgegenhaltungen:
- EP-A2- 1 447 062
- WO-A1-01/43669
- DE-A1-102007 053 389
- DE-A1-102008 008 284

## Beschreibung

Die Erfindung betrifft ein Verfahren und einer Vorrichtung zur Steuerung eines künstlichen orthetischen oder prothetischen Gelenkes einer unteren Extremität mit einer Widerstandseinrichtung, der zumindest ein Aktuator zugeordnet ist, über den der Beugeund/oder Streckwiderstand in Anhängigkeit von Sensordaten verändert wird, wobei während der Benutzung des Gelenkes über Sensoren Zustandsinformationen bereitgestellt werden. Die Vorrichtung und das Verfahren sind insbesondere zur Steuerung eines orthetischen oder prothetischen Kniegelenkes geeignet, jedoch sind auch Hüft- oder Knöchelgelenke der Erfindung zugänglich.

Künstliche Gelenke, insbesondere Kniegelenke, für Orthesen oder Prothesen weisen ein oberes Anschlussteil und ein unteres Anschlussteil auf, die über eine Gelenkeinrichtung miteinander verbunden sind. An dem oberen Anschlussteil sind im Falle eines Kniegelenkes Aufnahmen für einen Oberschenkelstumpf oder eine Oberschenkelschiene angeordnet, während an dem unteren Anschlussteil ein Unterschenkelschaft oder eine Unterschenkelschiene angeordnet ist. Im einfachsten Fall ist das obere Anschlussteil mit dem unteren Anschlussteil durch ein einachsiges Gelenk verschwenkbar miteinander verbunden. Eine solche Anordnung ist nur in Ausnahmefällen ausreichend, um den gewünschten Erfolg, beispielsweise eine Unterstützung bei dem Einsatz einer Orthese oder ein natürliches Gangbild bei dem Einsatz in einer Prothese, zu gewährleisten.

Um die unterschiedlichen Anforderungen während der verschiedenen Phasen eines Schrittes oder bei anderen Verrichtungen möglichst natürlich darzustellen oder zu unterstützen, sind Widerstandseinrichtungen vorgesehen, die einen Flexionswiderstand oder einen Extensionswiderstand bereitstellen. Über den Flexionswiderstand wird eingestellt, wie leicht das untere Anschlussteil gegenüber dem oberen Anschlussteil in Flexionsrichtung verschwenkbar ist. Bei einem Kniegelenk wird daher über den Flexionswiderstand eingestellt, wie leicht der Unterschenkelschaft oder die Unterschenkelschiene im Verhältnis zu dem Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und kann einen Streckanschlag ausbilden. Bei anderen Gelenktypen, wie dem Hüftgelenk oder dem Knöchelgelenk, gelten diese Ausfiihrungen entsprechend den kinematischen Verhältnissen.

Über einstellbare Widerstandseinrichtungen ist es möglich, den jeweiligen Flexionsund/oder Extensionswiderstand an den Nutzer der prothetischen oder orthetischen Einrichtung anzupassen oder auf verschiedene Gang- oder Bewegungssituationen einzugehen, um bei sich verändernden Bedingungen einen angepassten Widerstand bereitstellen zu können.

Aus der DE 10 2008 008 284 A1 ist ein orthopädietechnisches Kniegelenk mit einem Oberteil und einen verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von den ermittelten Sensordaten eingestellt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für eine Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2007 053 389 A1 beschreibt ein Verfahren und eine Vorrichtung zur Steuerung eines orthopädischen Gelenkes einer unteren Extremität mit zumindest einem Freiheitsgrad mit einem verstellbaren Aktuator zur Anpassung einer orthopädischen Einrichtung, die oberseitige Anschlussmittel an eine Gliedmaße und ein distal zu Anschlussmitteln gelenkig angeordnetes orthopädisches Gelenk aufweist, an Gehsituationen, die von einem Gehen in der Ebene abweichen. Dabei werden mehrere Parameter der orthopädischen Einrichtung über Sensoren erfasst, die erfassten Parameter mit Kriterien, die anhand mehrerer Parameter und/oder Parameterverläufe erstellt worden und in einer Rechnereinheit abgelegt sind, verglichen und ein Kriterium ausgewählt, das anhand der ermittelten Parameter oder Parameterverläufe geeignet ist. Auf der Grundlage des gewählten Kriteriums werden Beugungswiderstände, Bewegungsumfänge, Antriebskräfte und/oder deren Verläufe eingestellt, um Sonderfunktionen zu steuern, die vom Gehen in der Ebene abweichen. Ein Kippwinkel eines Teils der orthopädischen Einrichtung im Raum und/oder ein Verlauf einer Kippwinkeländerung eines Teils der orthopädischen Einrichtung können als Parameter herangezogen werden.

Die EP 1237513 B1 beschreibt eine Prothese oder Orthese mit einer Steuerungseinrichtung und einem damit gekoppelten Sensor, der einen Neigungswinkel bezogen auf eine feststehende Linie eines mit einem Gelenk verbundenen Teils erfasst. Auf der Basis der Neigungswinkeldaten werden die Bewegungseigenschaften des Gelenks verändert, also das Gelenk abgebremst oder freigegeben.

Es hat sich bewährt, dass ein Kniegelenk in der Standphase während des Gehens oder auch während des Stehens einen hohen Widerstand bietet, wobei das Kniegelenk nicht vollständig gesperrt wird. In diesem Fall wird das Beugen des Gelenks im Stehen dadurch verhindert, dass der Kraftvektor vor der Gelenkachse liegt und somit das Gelenk in den Streckanschlag drückt.

Ein Nichtsperren des Gelenkes in der Stehsituation hat den Vorteil, dass der Nutzer noch Eingriffsmöglichkeiten in die Gelenksbewegung hat. Sollte er zum Beispiel auf einer Treppe stehen und das Gleichgewicht verlieren, würde er über ein gesperrtes Gelenk unkontrolliert stürzen, während er ein Gelenk mit einem hohen Widerstand mittels der Stumpfkraft noch beugen kann. Dadurch können die Folgen eines Sturzes vermindert oder das Stürzen an sich verhindert werden. Auch erleichtert der Widerstand das Manövrieren des Gelenkes in engen Räumen und das Niedersetzen. Das Niedersetzen wird dadurch erleichtert, das der Widerstand eine Unterstützung gegen ein zu rasches Einsinken bietet. Der Widerstand, der gegen das zu rasche Einsinken aufgebracht wird, müsste anderenfalls durch die nicht versorgte Seite, also das gesunde Bein, aufgebracht werden.

Befindet sich ein Prothesen- oder Orthesennutzer in einer sitzenden Stellung, kann die Situation auftreten, dass der Flexionswiderstand und/oder Extensionswiderstand so hoch eingestellt ist, dass ein angenehmes Sitzen nicht möglich ist. Während des Sitzens ist es häufig notwendig und angenehm, dass die freie Beweglichkeit des Gelenkes vorhanden ist, um die beim Sitzen durchgeführten kleinen Bewegungsumfänge ohne Probleme ausführen zu können.

Die EP 1 447 062 A2 betrifft eine Beinprothese zur Anpassung an einen Oberschenkelstumpf mit einem Adapter für einen daran befestigtes Kniegelenk, an dem ein Prothesenunterschenkel und Prothesenfuß befestigt sind. Das Kniegelenk ist dergestalt ausgebildet, dass es beim Übergang von einer Strecklage in eine Beugelage eine kombinierte Roll-Gleitbewegung ausführt. In jeder Beugeposition des Kniegelenkes wird durch einen Wandler ein eindeutiges elektrisches Signal erzeugt, das einer programmierbaren Steuerungseinrichtung zugeführt wird, die ein Signal generiert, mit dem ein elektrisch verstellbarer Aktuator angesteuert wird. In Abhängigkeit des Signals wird der Widerstand des Kniegelenks gegen eine weitere Beugung vergrößert bzw. verringert.

Die WO 01/43669 A1 betrifft eine Vorrichtung zum Ersetzen eines Körpergliedes oder der Funktion eines Körpergliedes mit zumindest zwei Teilen, die über ein künstliches Gelenk miteinander verbunden sind. Eine Steuerungsvorrichtung für das Gelenk ist vorgesehen, die mit einem Sensor gekoppelt ist, der die Position eines Teils, das mit dem Gelenk verbunden ist, in Relation zu einer feststehenden Linie ermittelt. Die Steuerungsvorrichtung ist eingerichtet, um das Gelenk auf der Basis der von dem Sensor ermittelten Positionsdaten zu beeinflussen.

Aufgabe der vorliegenden Erfindung ist es daher, eine Vorrichtung und ein Verfahren bereitzustellen, mit denen es möglich ist, einen sitzenden Zustand zu erkennen und eine Anpassung des Widerstandes des Gelenkes an die Sitzsituation vorzunehmen.

Erfindungsgemäß wird diese Aufgabe durch ein Verfahren mit den Merkmalen des Hauptanspruches und eine Vorrichtung mit den Merkmalen des nebengeordneten Anspruchs gelöst. Vorteilhafte Ausgestaltungen und Weiterbildungen sind in den Unteransprüchen aufgeführt.

Das erfindungsgemäße Verfahren sieht vor, dass zur Steuerung eines künstlichen orthetischen oder prothetischen Gelenkes einer unteren Extremität eine Widerstandseinrichtung vorgesehen ist, der zumindest ein Aktuator zugeordnet ist, über den der Beuge- und/oder Streckwiderstand im Abhängigkeit von Sensordaten verändert wird. Dabei werden während der Benutzung des Gelenkes über Sensoren Zustandsinformationen bereitgestellt. Der Inertialwinkel eines Oberschenkelteils wird gemessen und der Widerstand reduziert, wenn das Oberschenkelteil wenigstens 45°, insbesondere wenigstens 70° zur Vertikalen aufweist und/oder der Kniewinkel größer als 45°, insbesondere größer als 80° ist. Als Vertikale wird dabei die Gravitationsrichtung angesehen, während der Kniewinkel derjenige Winkel ist, der zwischen dem oberen Anschlussteil und dem unteren Anschlussteil des Kniegelenkes vorhanden ist, ausgehend von einer gestreckten Stellung, in der der Kniewinkel 0° beträgt. Je größer die Beugung des unteren Anschlussteils gegenüber dem oberen Anschlussteil ist, desto größer wird der Kniewinkel. Wird der Sitzzustand erkannt, lässt sich die Prothese oder Orthese leichter manövrieren, wenn der Widerstand, vorzugsweise sowohl in Extensions- als auch in Flexionsrichtung, reduziert wird, ggf. kann die Widerstandeinrichtung so verändert werden, dass der zusätzliche Widerstand gegen Null geht. Das Sitzen wird dadurch erkannt, dass der Inertialwinkel des Oberschenkelteils, also der Absolutwinkel des Oberschenkelteils relativ zu der Vertikalen, gemessen wird. Ist der Inertialwinkel des Oberschenkelteil im Wesentlichen waagerecht, also wenigstens 70° zur Vertikalen, kann davon ausgegangen werden, dass ein Sitzen vorliegt oder kurz bevor steht. Darüber hinaus kann alternativ oder ergänzend der Kniewinkel zu Beurteilung des Sitzzustandes herangezogen werden. Sofern der Kniewinkel größer als 80° ist, kann davon ausgegangen werden, dass eine sitzende Stellung eingenommen ist. Der Kniewinkel kann auch allein als maßgebliche Größe zur Steuerung herangezogen werden, ebenso kann der Inertialwinkel des Oberschenkelteils alleine ausreichen, um eine Sitzstellung zu ermitteln.

Eine Variante der Erfindung sieht vor, dass der Widerstand bei Erreichen oder Unterschreiten eines Schwellwertes für die Bodenreaktionskraft reduziert wird. Dazu ist es vorgesehen, dass die Bodenreaktionskraft auf die Prothese oder Orthese gemessen wird. Erreicht oder unterschreitet die Bodenreaktionskraft den festgelegten Schwellwert, da aufgrund der Sitzposition nur ein geringer Teil der sonst üblichen Gewichtskraft auf eine der Komponenten, beispielsweise das Unterschenkelteil, das Gelenk oder den Prothesenfuß ausgeübt wird, kann der Widerstand reduziert werden.

Es ist möglich und vorgesehen, dass der Widerstand nach Erreichen des Schwellwertes von beispielsweise wenigstens 45° für den Inertialwert des Oberschenkelteils und/oder des Kniewinkels kontinuierlich mit zunehmendem Winkel reduziert wird. Dies dient als Hinsetzhilfe, sodass der Prothesen- und Orthesennutzer zunächst durch einen hohen Flexionswiderstand nicht das gesamte Gewicht über das nicht versorgte Bein abfangen muss. Befindet sich beispielsweise das Oberschenkelteil in Winkelstellung von 45° zur Vertikalen, kann kontinuierlich der Flexionswiderstand verringert werden, sodass sich leichter hingesetzt werden kann.

Der Inertialwinkel des Oberschenkelteils kann unmittelbar gemessen werden, also durch eine entsprechende Sensoreinrichtung an einem Oberschenkelteil, oder aus dem Inertialwinkel eines Unterschenkelteils und einem Gelenkwinkel, der zwischen dem Unterschenkelteil und dem Oberschenkelteil besteht, errechnet werden.

Eine Weiterbildung der Erfindung sieht vor, dass der Widerstand nur dann reduziert wird, wenn das Oberschenkelteil sich für einen bestimmten Zeitraum in der geneigten Position befindet, also ab einem Winkel von ca. 45°, insbesondere 70° zur Vertikalen. Dadurch wird verhindert, dass bei einem kurzfristigen Erreichen der angenähert waagerechten Position des Oberschenkelteils eine schlagartige oder auch kontinuierliche Verringerung des Flexionswiderstandes und/oder des Extensionswiderstandes erfolgt. Es ist somit ein Zeitglied vorhanden, das erst nach einem bestimmten Zeitablauf eine Reduzierung des Widerstandes ermöglicht. Die zeitgesteuerte Veränderung des Widerstandes in Abhängigkeit von der Dauer der nahezu waagerechten Position des Oberschenkelteils bietet eine zusätzlich Sicherheit. Eine entsprechende zeitgesteuerte Veränderung kann auch auf Basis des Kniewinkels erfolgen, sodass die Widerstandsreduzierung erst dann durchgeführt wird, wenn der Kniewinkel über einen bestimmten Zeitraum hinweg einen bestimmten Mindestwert aufweist.

Eine Weiterbildung der Erfindung sieht vor, dass der Widerstand, insbesondere der Flexionswiderstand, nach der Reduktion in Abhängigkeit einer Gelenkwinkeländerung und/oder einer Inertialwinkeländerung erhöht wird, um eine Aufstehhilfe bereitzustellen. Wird ein Nutzer der Prothese oder Orthese aufstehen, besteht die Gefahr, dass die Stehposition nicht ohne weiteres erreicht wird. Um ein Zurückfallen in die Sitzposition ohne eine Widerstandseinrichtung zu verhindern, wird detektiert, ob sich der Gelenkwinkel und/oder der Inertialwinkel verändert, insbesondere in Richtung Streckung des Gelenkes und Annäherung des Oberschenkelteils an die Vertikale. Ist dies der Fall, wird der Widerstand, insbesondere der Flexionswiderstand, erhöht.

Eine Verringerung des Widerstandes kann unterbleiben, wenn eine Gelenkwinkelveränderung ermittelt wird, also wenn sich die Einrichtung nicht in einem statischen oder einem angenähert statischen Zustand befindet. Auf diese Weise ist es möglich, eine Aufstehhilfe oder eine Hinsetzhilfe zu realisieren.

Ist das Sitzen detektiert und der Widerstand verringert worden, kann die Steuerung in einen Energiesparmodus fallen, in dem beispielsweise die Taktung verlangsamt oder einzelne Sensorwerte nicht abgefragt werden, bis sich auf Grund anderer Sensorwerte ergibt, dass die sitzende Stellung verlassen wird. Ebenfalls können nicht genutzte Verbraucher in einen Stand-by-Zustand geschaltet werden, so dass insgesamt der Energieverbrauch reduziert wird.

Zur Bereitstellung einer erhöhten Sicherheit können mehrere Steueralgorithmen vorhanden sein, die auf der Grundlage von Messwerten unterschiedlicher Einrichtungen zur Erfassung von Winkeln und Kräften arbeiten, so dass bei Ausfall einer Einrichtung zur Erfassung von Winkeln und Kräften die übrigen Messwerte zur Steuerung der Veränderung des Streckund/oder Beugewiderstandes verwendet werden können. Dadurch ist es möglich, dass eine Redundanz aufgebaut wird. Die Algorithmen können mit Messwerten arbeiten, die von unterschiedlichen Gruppen von Einrichtungen zur Erfassung von Winkeln und Kräften stammen, wobei es in den Gruppen einzelne Überschneidungen geben kann, so dass bei Ausfall einer Einrichtung zur Erfassung von Winkeln und Kräften die übrigen Messwerte zur Steuerung der Veränderung des Streck- und/oder Beugewiderstandes über andere Algorithmen verwendet werden können. Durch die Redundanz der Steuerungsmöglichkeiten ergibt sich eine erhöhte Sicherheit gegen einen Ausfall der Prothesen oder Orthesen.

Die erfindungsgemäße Vorrichtung zur Durchführung des vorgeschriebenen Verfahrens sieht vor, dass eine einstellbare Widerstandseinrichtung vorhanden ist, die zwischen zwei gelenkig aneinander gelagerten Komponenten eines künstlichen orthetischen oder prothetischen Gelenkes einer unteren Extremität angeordnet ist, wobei eine Steuereinrichtung und Sensoren vorhanden sind, die einen Inertialwinkel einer Komponente und/oder einen Gelenkwinkel der Vorrichtung erfassen. Es ist eine Einstellvorrichtung vorgesehen, über die eine lageabhängige Widerstandsänderung aktivierbar und/oder deaktivierbar ist, um die Sonderfunktion des Sitzens und die auf die Erkennung des Sitzens folgenden Reduzierungen des Widerstandes aktiv einschalten oder ausschalten zu können.

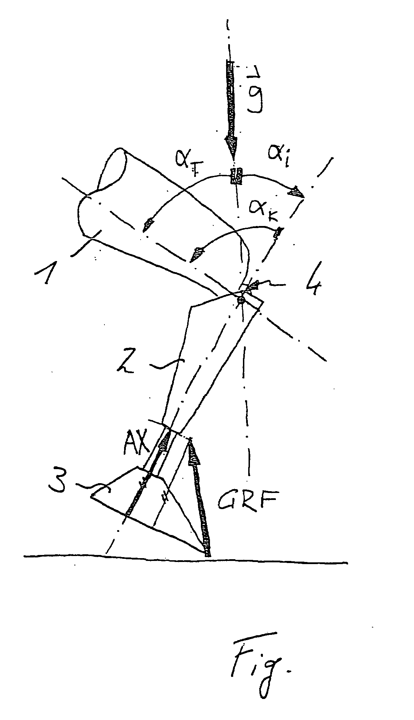

Nachfolgend wird ein Ausführungsbeispiel der Erfindung anhand der beigefügten Figur näher erläutert. In der einzigen Figur ist schematisch eine Prothese in sitzender Position dargestellt. Die Prothese weist ein Oberschenkelteil 1 und ein Unterschenkelteil 2 auf, die über ein Prothesenkniegelenk 4 schwenkbar miteinander verbunden sind. An dem Unterschenkelteil 2 ist ein Prothesenfuß 3 angeordnet. Ebenfalls sind in dem Unterschenkelteil 2 eine Widerstandseinrichtung sowie ein Aktuator angeordnet, der aufgrund von Sensordaten, die über eine Steuereinheit ausgewertet werden, den Extensions- oder Flexionswiderstand gegen eine Extensions- oder Flexionsbewegung des Oberschenkelteils 1 relativ zu dem Unterschenkelteil 2 einstellt. Befindet sich der Prothesennutzer in einer sitzenden Position, ist es angenehm, wenn der Extensionswiderstand und der Flexionswiderstand der Widerstandseinrichtung gering sind, so dass die während des Sitzens ausgeführten Bewegungen, die in der Regel einen geringen Bewegungsumfang aufweisen, ohne Beeinträchtigungen durchgeführt werden können.

Um die Widerstandsverringerung automatisch durchführen zu können, ist es vorgesehen, dass der sitzende Zustand detektiert wird. Dazu ist es vorgesehen, dass der Inertialwinkel α_{τ} und/oder der Kniewinkel α_{κ} gemessen werden. Der Inertialwinkel α_{τ} des Oberschenkelteils 1 wird zur Vertikalen gemessen, die in Schwerkraftrichtung wirkend angenommen wird. In der Figur ist dies durch den Schwerkraftvektor g angedeutet. Als Bezugsgröße für den Inertialwinkel α_{τ} wird die Längsachse des Oberschenkelteils 1 angenommen, die durch die Schwenkachse des Prothesenkniegelenkes 4 geht. Dabei entspricht die Längsachse ungefähr der Orientierung eines natürlichen Oberschenkelknochens und erstreckt sich im Wesentlichen mittig zu dem Oberschenkelteil 1, das in der Regel als ein Oberschenkelschaft ausgebildet ist.

Der Kniewinkel α_{κ} liegt zwischen der Längsstreckung des Unterschenkelteils 2 und der Längserstreckung des Oberschenkelteils 1 an. Auch hier geht die Längserstreckung des Unterschenkelteils 2 durch die Gelenkachse des Prothesenkniegelenkes 4. Der Inertialwinkel α_{τ} des Oberschenkelteils 1 kann sich aus dem Inertialwinkel αᵢ des Unterschenkelteils 2 und dem Kniewinkel α_{κ} errechnen lassen, wobei sich nach der Figur Inertialwinkel α_{τ} des Oberschenkelteils 1 aus der Differenz zwischen dem Kniewinkel α_{κ} und dem Inertialwinkel αᵢ des Unterschenketeils 2 ergibt.

Darüber hinaus kann die Bodenreaktionskraft GRF bzw. die Axialkraft AX, die den Anteil der Bodenreaktionskraft in Richtung des Unterschenkelteils 2 darstellt, bestimmt werden, um auf Grundlage der vorliegenden Kräfte zu entscheiden, ob sich der Prothesennutzer in einer sitzenden Stellung befindet oder nicht.

Es ist vorgesehen, dass die Widerstandsreduzierung dann stattfindet, wenn das Oberschenkelteil 1 wenigstens einen Inertialwinkel α_{τ} von wenigstens 45°, insbesondere wenigstens 70° aufweist und/oder der Kniewinkel α_{κ} größer als 45°, insbesondere größer als 80° ist. In der Regel kann bei dem Eintreten eines oder mehrerer solcher Winkelgrößen davon ausgegangen werden, dass sich der Prothesennutzer in einer sitzenden Stellung befindet. Ergänzt kann dieses Kriterium dadurch werden, dass die Bodenreaktionskraft GRF gemessen wird. In der Regel reduziert sich die Bodenreaktionskraft GRF signifikant, wenn der Prothesennutzer sitzt. Unterschreitet also die Bodenreaktionskraft GRF einen Schwellwert, ist dies ein weiterer Faktor bei der Beurteilung, ob ein sitzender Zustand erreicht ist oder nicht.

Eine schlagartige Reduzierung des Widerstandes nach Erreichen bestimmter Schwellwerte wird häufig als nicht angenehm empfunden. Daher ist es vorgesehen, dass der Widerstand nach Erreichen eines Schwellwertes für den Inertialwinkel α_{τ} des Oberschenkelteils 1 und/oder des Kniewinkels α_{κ} kontinuierlich mit zunehmendem Inertialwinkel α_{τ} und/oder Kniewinkel α_{κ} reduziert wird. Dadurch wird das sich Hinsetzen unterstützt und der Übergang von dem Stehen zu dem Sitzen erleichtert. Der Inertialwinkel α_{τ} des Oberschenkelteils 1 kann unmittelbar über einen entsprechenden Sensor ermittelt werden, alternativ ist es vorgesehen, dass der Inertialwinkel α_{τ} des Oberschenkelteils aus dem Inertialwinkel αᵢ des Unterschenkelteils 2 und dem Kniewinkel α_{κ} ermittelt wird.

Befindet sich das Oberschenkelteil 1 über einen bestimmten Zeitraum in einer geneigten Stellung, also in einem im Wesentlichen wagerechten Zustand, so dass der Inertialwinkel α_{τ} zwischen beispielsweise 70° und 110° befindlich ist, kann nach Ablauf einer vorgegebenen Zeit ebenfalls eine Reduzierung des Widerstandes der Widerstandseinrichtung erfolgen, da dann angenommen werden kann, dass der Prothesennutzer sitzt oder sich in absehbarer Zeit nicht erneut erheben wird.

Verändert sich der Gelenkwinkel α_{κ} oder der Inertialwinkel α_{τ} des Oberschenkelteils 1, kann der Widerstand wieder erhöht werden, bevorzugt kontinuierlich erhöht werden, damit eine Rückfallsicherung vorhanden ist, um dem Patienten das Aufstehen zu erleichtern. Weiterhin kann es vorgesehen sein, dass eine Verringerung des Widerstandes unterbleibt, auch wenn die Winkelstellungen vorhanden sind, so lange der Patient das Bein bewegt, also wenn eine Gelenkwinkeländerung beispielsweise des Kniewinkels α_{κ} detektiert wird.

## Patentansprüche

1. Verfahren zur Steuerung eines künstlichen orthetischen oder prothetischen Gelenkes (4) einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes (4) über Sensoren Zustandsinformationen bereitgestellt werden, **dadurch gekennzeichnet, dass** der Inertialwinkel α_{τ} eines Oberschenkelteils (1) gemessen und der Widerstand reduziert wird, wenn das Oberschenkelteil (1) wenigstens 45° zur Vertikalen und/oder der Kniewinkel α_{κ} größer 45° sind.

2. Verfahren nach Anspruch 1, **dadurch gekennzeichnet, dass** der Widerstand bei Erreichen oder Unterschreiten eines Schwellwertes für die Bodenreaktionskraft reduziert wird.

3. Verfahren nach Anspruch 1 oder 2, **dadurch gekennzeichnet, dass** der Widerstand nach Erreichen eines Schwellwertes für den Inertialwinkel α_{τ} des Oberschenkelteils (1) und/oder Kniewinkels α_{κ} kontinuierlich mit zunehmenden Inertialwinkel α_{τ} und/oder Kniewinkel α_{κ} reduziert wird.

4. Verfahren nach einem der voranstehenden Ansprüche, **dadurch gekennzeichnet, dass** der Inertialwinkel α_{τ} des Oberschenkelteiles (1) unmittelbar oder aus dem Inertialwinkel α_{τ} eines Unterschenkelteiles und einem Gelenkwinkel α_{κ} ermittelt wird.

5. Verfahren nach einem der voranstehenden Ansprüche, **dadurch gekennzeichnet, dass** der Widerstand reduziert wird, wenn das Oberschenkelteil (1) sich für einen vorbestimmten Zeitraum in der geneigten Position befindet.

6. Verfahren nach einem der voranstehenden Ansprüche, **dadurch gekennzeichnet, dass** der Widerstand nach der Reduzierung in Abhängigkeit von einem gemessenen Gelenkwinkel α_{κ} und/oder einem Inertialwinkel α_{τ} des Oberschenkelteils (1) erhöht wird.

7. Verfahren nach einem der voranstehenden Ansprüche, **dadurch gekennzeichnet, dass** eine Verringerung des Widerstandes unterbleibt, wenn eine Gelenkwinkeländerung ermittelt wird.

8. Verfahren nach einem der voranstehenden Ansprüche, **dadurch gekennzeichnet, dass** nach dem Reduzieren des Widerstandes ein Energiesparmodus eingeschaltet wird.

9. Verfahren nach einem der voranstehenden Ansprüche, **dadurch gekennzeichnet, dass** mehrere Steueralgorithmen vorhanden sind, die auf der Grundlage von Messwerten unterschiedlicher Einrichtungen zur Erfassung von Winkeln und Kräften arbeiten, so dass bei Ausfall einer Einrichtung zur Erfassung von Winkeln und Kräften die übrigen Messwerte zur Steuerung der Veränderung des Streck- und/oder Beugewiderstandes verwendet werden.

10. Vorrichtung zur Durchführung des Verfahrens nach einem der voranstehenden Ansprüche, mit einer einstellbaren Widerstandseinrichtung, die zwischen zwei gelenkig aneinander gelagerten Komponenten (1, 2) eines künstlichen orthetischen oder prothetischen Gelenkes (4) einer unteren Extremität angeordnet ist, mit einer Steuereinrichtung und Sensoren, die einen Inertialwinkel α_{τ} einer Komponente und/oder einen Gelenkwinkel α_{κ} der Vorrichtung erfassen, **dadurch gekennzeichnet, dass** eine Einstellvorrichtung vorgesehen ist, über die eine lageabhängige Widerstandsänderung aktivierbar und/oder deaktivierbar ist.

## Claims

1. A method for controlling an artificial orthotic or prosthetic joint (4) of a lower extremity with a resistance device to which at least one actuator is assigned, via which actuator the flexion and/or extension resistance is changed depending on sensor data, with status information being made available via sensors during the use of the joint (4), **characterized in that** the inertial angle (α_{τ}) of a thigh part (1) is measured, and the resistance is reduced when the thigh part (1) is at least 45° to the vertical and/or the knee angle (α_{κ}) is greater than 45°.

2. The method as claimed in claim 1, **characterized in that** the resistance is reduced when the ground reaction force reaches or is below a threshold value.

3. The method as claimed in claim 1 or 2, **characterized in that**, after reaching a threshold value for the inertial angle (α_{τ}) of the thigh part (1) and/or knee angle (α_{κ}), the resistance is reduced continuously with increasing inertial angle (α_{τ}) and/or knee angle (α_{κ}).

4. The method as claimed in one of the preceding claims, **characterized in that** the inertial angle (α_{τ}) of the thigh part (1) is determined directly or from the inertial angle (α_{τ}) of a lower leg part and a joint angle (α_{κ}).

5. The method as claimed in one of the preceding claims, **characterized in that** the resistance is reduced when the thigh part (1) is located in the inclined position for a predefined period of time.

6. The method as claimed in one of the preceding claims, **characterized in that**, after being reduced, the resistance is increased as a function of a measured joint angle (α_{κ}) and/or an inertial angle (α_{τ}) of the thigh part (1).

7. The method as claimed in one of the preceding claims, **characterized in that** a reduction in resistance is suppressed if a change of joint angle is determined.

8. The method as claimed in one of the preceding claims, **characterized in that**, after the resistance has been reduced, an energy-saving mode is switched on.

9. The method as claimed in one of the preceding claims, **characterized in that** a plurality of control algorithms are present which, on the basis of measured values of different devices for detecting angles and forces, operate in such a way that, if a device for detecting angles and forces fails, the other measured values are used to control the change in the extension and/or flexion resistance.

10. An appliance for carrying out the method as claimed in one of the preceding claims, with an adjustable resistance device, which is arranged between two mutually articulated components (1,2) of an artificial orthotic or prosthetic joint (4) of a lower extremity, and with a control device and sensors, which detect an inertial angle (α_{τ}) of a component and/or a joint angle (α_{κ}) of the appliance, **characterized in that** an adjustment device is provided, by means of which a position-dependent change of resistance can be activated and/or deactivated.

## Revendications

1. Procédé pour la commande d'une articulation orthétique ou prothétique artificielle (4) d'une extrémité inférieure avec un système à résistance auquel est associé au moins un actionneur, via lequel la résistance à la flexion et/ou à l'extension est modifiée en fonction de données sensorielles, dans lequel on prépare des informations d'état via des capteurs pendant l'utilisation de l'articulation (4),
**caractérisé en ce que** l'angle d'inertie α_{τ} d'une partie de cuisse (1) est mesuré et la résistance est réduite quand la partie de cuisse (1) est au moins à 45° par rapport à la verticale et/ou l'angle de genou α_{κ} est supérieur à 45°.

2. Procédé selon la revendication 1, **caractérisé en ce que** la résistance est réduite lorsqu'on atteint ou lorsqu'on passe au-dessous d'une valeur seuil pour la force de réaction au sol.

3. Procédé selon la revendication 1 ou 2, **caractérisé en ce que** la résistance est réduite lorsqu'on atteint une valeur seuil pour l'angle d'inertie α_{τ} de la partie de cuisse (1) et/ou l'angle de genou α_{κ} est continûment réduit lorsque que l'angle d'inertie α_{τ} augmente, et/ou l'angle de genou α_{κ} est réduit.

4. Procédé selon l'une des revendications précédentes, **caractérisé en ce que** l'angle d'inertie α_{τ} de la partie de cuisse (1) est déterminé soit directement soit à partir de l'angle d'inertie α_{τ} d'une partie de jambe et d'un angle d'articulation α_{κ}.

5. Procédé selon l'une des revendications précédentes, **caractérisé en ce que** la résistance est réduite quand la partie de cuisse (1) se trouve pendant une durée prédéterminée dans la position inclinée.

6. Procédé selon l'une des revendications précédentes, **caractérisé en ce que**, après la réduction, la résistance est augmentée en fonction d'un angle d'articulation mesuré α_{κ} et/ou d'un angle d'inertie α_{τ} de la partie de cuisse (1).

7. Procédé selon l'une des revendications précédentes, **caractérisé en ce qu'**il n'y a pas de réduction de la résistance lorsqu'on détermine une variation angulaire de l'articulation.

8. Procédé selon l'une des revendications précédentes, **caractérisé en ce que**, après la réduction de la résistance, on met en service un mode d'économie d'énergie.

9. Procédé selon l'une des revendications précédentes, **caractérisé en ce qu'**il est prévu plusieurs algorithmes de commande qui fonctionnent sur la base de valeurs de mesures de différents systèmes pour la détection des angles et des forces, de sorte que lors de la défaillance d'un système pour la détection des angles et les forces, les autres valeurs de mesure sont utilisées pour la commande de la modification de la résistance à l'extension et/ou à la flexion.

10. Appareil pour la mise en oeuvre du procédé selon l'une des revendications précédentes, comprenant un système à résistance réglable, qui est agencé entre deux composants (1, 2) montés de façon articulée l'un sur l'autre d'une articulation orthétique ou prothétique artificielle (4) d'une extrémité inférieure, comprenant un système de commande et des capteurs qui détectent un angle d'inertie α_{τ} d'un composant et/ou un angle d'articulation α_{κ} de l'appareil, **caractérisé en ce qu'**il est prévu un dispositif de réglage au moyen duquel une modification de la résistance en fonction de la position est susceptible d'être activée et/ou désactivée.
